# EUROPEAN PATENT SPECIFICATION

(11) **EP 2 331 189 B1**
(45) Date of publication and mention of the grant of the patent: **02.03.2016**
(21) Application number: 09812331.8
(22) Date of filing: 04.09.2009
(51) Int. Cl.: A61M 31/00, A61M 25/10, A61L 27/14, A61L 27/54, A61F 2/00, A61F 2/12, A61F 2/02, B29C 51/00, A61B 17/00, A61B 17/12, A61F 5/00, A61L 31/04, B29C 61/02, A61L 31/14, A61B 17/70, A61M 25/00, A61B 90/00

(54) **PROCESS FOR GENERATING MICROWALLED ENCAPSULATION BALLOONS**
VERFAHREN ZUR ERZEUGUNG MIKROWANDIGER VERKAPSELUNGSBALLONS
PROCÉDÉ DE PRODUCTION DE BALLONS D'ENCAPSULATION À MICROPAROIS

(30) Priority: 05.09.2008 US 191174 P
(43) Date of publication of application: 15.06.2011
(73) Proprietor: Cardiopolymers, Inc, Laguna Hills, CA 92653 (US)
(72) Inventor: SHANNON, Don, Trabuco Canyon CA 92679 (US); AHMANN, Frank, Laguna Hills CA 92653 (US)
(74) Representative: Nguyen Van Yen, Christian
(86) International application number: PCT/US2009/056123
(87) International publication number: WO 2010/028305

(56) References cited:
- EP-A2- 1 935 616
- WO-A1-98/33638
- WO-A2-2005/007873
- WO-A2-2007/140262
- US-A- 5 928 132
- US-A1- 2002 055 756
- US-A1- 2003 125 807
- US-A1- 2004 097 788
- US-A1- 2005 165 432
- US-A1- 2006 085 024

## Description

### BACKGROUND

### Field

The present disclosure relates to encapsulation balloons of biocompatible materials for emplacement within tissue, and to processes for preparing encapsulation balloons WO2007/140262, EP1935616 and WO2005/007873 disclose encapsulation ballons and for processes for multilayer films.

### SUMMARY

According to embodiments as defined in claim 1, disclosed is a process for generating a microwalled encapsulation balloon, comprising, in combination: providing a paddle insert having a top side and a bottom side; placing a balloon membrane further comprising a first membrane layer on at least a portion of the top side of the paddle insert and a second membrane layer on at least a portion of the bottom side of a paddle insert, such that portions of the first membrane layer and the second membrane layer extending beyond the paddle insert are in contact with each other; providing a first support layer on a top side of the first membrane layer and a second support layer on a bottom side of the second membrane layer; providing a first paddle die on a top side of the first support layer and a second paddle die on a bottom side of the second support layer; and pressing the first and second paddle dies together while applying heat to the balloon membrane.

Pressing the first and second paddle dies together may conform the balloon membrane to the paddle insert. Pressing may also compress the portions of the first and second membrane layers beyond the paddle insert to a high density state, whereby first and second membrane layers become an integrated balloon membrane. Pressing may also establish cohesive force between the first membrane layer to the first support layer and the second membrane layer to the second support layer.

Heat applied may be between about the melting point of the balloon membrane and the melting point of the support layers, or other temperatures between the melting points of disparate materials. The balloon membrane may be of at least one of PTFE, ePTFE, and FEP, or other polymers. The support layers and paddle insert may comprise a polyimide or other flexible, heat resistant material.

Upon removing the paddle insert, an interior portion of the balloon membrane may be provided and be accessible through an opening, whereby the first and second support layers maintain the shape of the balloon membrane achieved by pressing the first and second paddle dies together.

The process may further comprise removing the first and second paddle dies; and coupling the balloon membrane to a cannula by providing a cannula through the opening into the interior portion of the balloon membrane and applying a cylinder die to a portion of the balloon membrane that encloses the cannula.

The process may further comprise providing a dowel within a lumen of the cannula to the interior portion of the balloon membrane, such that the dowel extends beyond the distal end of the cannula, whereby the dowel maintains the opening of the interior portion of the balloon membrane as the cylinder die is applied.

### DRAWINGS

The above-mentioned features and objects of the present disclosure will become more apparent with reference to the following description taken in conjunction with the accompanying drawings wherein like reference numerals denote like elements and in which:
Figure 1A shows a perspective view showing an illustrative implementation of a system for compressing a balloon membrane around a paddle insert;
Figure 1B shows a cross-sectional view showing an illustrative implementation of a system for compressing a balloon membrane around a paddle insert;
Figure 1C shows a cross-sectional view showing an illustrative implementation of a system for compressing a balloon membrane around a paddle insert;
Figure 2A shows a cross-sectional view showing an illustrative implementation of a system for compressing a balloon membrane around a paddle insert;
Figure 2B shows a cross-sectional view showing an illustrative implementation of a system for compressing a balloon membrane around a paddle insert;
Figure 3A shows a perspective view showing an illustrative implementation of a system for coupling a balloon membrane to a cannula;
Figure 3B shows a cross-sectional view showing an illustrative implementation of a system for coupling a balloon membrane to a cannula;
Figure 3C shows a cross-sectional view showing an illustrative implementation of a system for coupling a balloon membrane to a cannula;
Figure 4 shows a cross-sectional view showing an illustrative implementation of a system for coupling a balloon membrane to a cannula;
Figure 5A shows a cross-sectional view showing an illustrative implementation of a balloon membrane with support layer coupled to a cannula;
Figure 5B shows a perspective view showing an illustrative implementation of a balloon membrane with support layer coupled to a cannula;
Figure 6A shows a cross-sectional view showing an illustrative implementation of a balloon membrane coupled to a cannula;
Figure 6B shows a perspective view showing an illustrative implementation of a balloon membrane coupled to a cannula;
Figure 7A illustrates in a cut-away view a distal portion of an exemplary implementation of the emplacement apparatus in a first operational condition;
Figure 7B illustrates in a cut-away view a distal portion of an exemplary implementation of the emplacement apparatus in a second operational condition;
Figure 7C illustrates in a cut-away view a distal portion of an exemplary implementation of the emplacement apparatus in a third operational condition;
Figure 7D illustrates in a cut-away view a distal portion of an exemplary implementation of the emplacement apparatus in a fourth operational condition;
Figure 7E illustrates in a cut-away view a distal portion of an exemplary implementation of the emplacement apparatus in a fifth operational condition;
Figure 8A illustrates in a cut-away view a distal portion of an exemplary implementation of the emplacement apparatus in a sixth operational condition;
Figure 8B illustrates in a cut-away view a distal portion of an exemplary implementation of the emplacement apparatus in a seventh operational condition;
Figure 8C illustrates in a cut-away view a distal portion of an exemplary implementation of the emplacement apparatus in an eighth operational condition;
Figure 8D illustrates in a cut-away view a distal portion of an exemplary implementation of the emplacement apparatus in a ninth operational condition;
Figure 9A illustrates in a cut-away view a distal portion of an exemplary implementation of the emplacement apparatus in an alternative sixth operational condition;
Figure 9B illustrates in a cut-away view a distal portion of an exemplary implementation of the emplacement apparatus in an alternative seventh operational condition;
Figure 9C illustrates in a cut-away view a distal portion of an exemplary implementation of the emplacement apparatus in an alternative eighth operational condition;
Figure 9D illustrates in a cut-away view a distal portion of an exemplary implementation of the emplacement apparatus in an alternative ninth operational condition;
Figure 10 shows a perspective view showing an illustrative implementation of a capsule;
Figure 11 shows a perspective view showing another illustrative implementation of a capsule; and
Figure 12 shows a perspective view showing yet another illustrative implementation of a capsule.

### DETAILED DESCRIPTION

In various treatment regimens it is advantageous to inject various types of flowable material within tissue of an organ of the body. However, injection of such material within tissue presents certain problems. In the case of a polymer, for example, the polymer may properly cross-link and form a mass within the tissue, but then degrade at such a rate that the therapeutic effect is lost before the therapeutic effect has been entirely realized. The polymer may diffuse within the interstitial spaces of the tissue before cross-linking, which, while desirable in some applications, may not be desirable in other applications.

According to examples, the present application may be applied in a system and method for volume augmentation of tissue in a living being, such as a human. The system consists generally of an encapsulation balloon used as a tissue-filling device and a method for delivering said tissue-filling device into tissue. The tissue-filling device consists of fill material and an enclosing membrane. Preferably, the enclosing membrane forms a container that may be filled.

According to examples, microwalled encapsulation balloons may be produced for use in the body of a patient. Encapsulation balloons may be biocompatible and provide a variety of functional benefits when emplaced in a patient. For example, emplaced encapsulation balloons may fill a space within the tissue of a patient. The techniques described herein are applicable to many tissues of the body. In cardiac applications, for example, depending on the therapeutic effect sought, a capsule may be emplaced in a single injection site in healthy or diseased tissue of the myocardium, or capsules may be injected into injection sites dispersed over a region of the myocardium or arranged along the myocardium in a therapeutically effective pattern. The techniques described herein are applicable, for example, to the injection of polymer within the ventricular wall of the heart, as described in United States Patent Application Publication No. US 2005/0271631 dated December 8, 2005 (Randall J. Lee et al., Material Compositions and Related Systems and Methods for Treating Cardiac Conditions), and in United States Patent Application Publication No. US 2008/0065046 dated March 13, 2008 (Hani N. Sabbah et al., Intramyocardial Patterning for Global Cardiac Resizing and Reshaping). When so emplaced, the polymer reduces stress within the ventricular wall and, over time, allows the heart to recover functionality. Encapsulation of the polymer within the ventricular wall prevents the polymer from being absorbed by the body before the heart recovers functionality.

An encapsulation balloon may be made of any biologically compatible material that is capable of being placed into a collapsed condition and inflatable from the collapsed condition into an inflated condition without damage or rupture to the balloon. Examples of suitable balloon materials include polytetrafluoroethylene ("PTFE"), expanded polytetrafluoroethylene ("ePTFE"), fluorinated ethylene propylene ("FEP"), low density polyethylene ("LDPE"), polypropylene, polyvinyl chloride ("PVC"), polydimethylsiloxane, polyethylene terephthalate ("PET") (Dacron®), polyamides (nylon), polyether urethane, polycarbonate, polysulfones, polymethyl methacrylate, poly 2-hydroxy-ethylmethacrilate (PHEMA), and so forth. The material of the encapsulation balloon may be porous where tissue ingrowth into the encapsulation balloon and/or controlled diffusion of substances from the biomaterial through the encapsulation balloon into the tissue is desired. An example of a suitable porous material is PTFE. The material of the encapsulation balloon may be substantially impermeable where biomaterial is to be securely contained by the encapsulation balloon. An example of a suitable impermeable material is FEP.

According to of the present disclosure, a process for generating a microwalled encapsulation balloon is disclosed, wherein the encapsulation balloon may contain a variety of customizable and selectable characteristics based on applications thereof. The product produced by processes and examples disclosed herein may be biocompatible and facilitate various types of treatments for a patient.

According to examples, a two or more membrane layers may be provided to be combined as an integrated balloon membrane of an encapsulation balloon. The balloon membrane may provide a hollow and fillable interior portion that provides selectable volume control of the encapsulation balloon. An opening may be provided to allow access to the interior portion for inflation of the encapsulation balloon upon delivery into a patient.

According to examples, a balloon membrane of an encapsulation balloon may be biocompatible and provide various features for emplacement into a patient. For example, selectable porosities may be achieved to encourage tissue ingrowth at the emplacement location of an encapsulation balloon. Various sizes and geometries may also be achieved based on the need for certain characteristics at and beyond the time of emplacement.

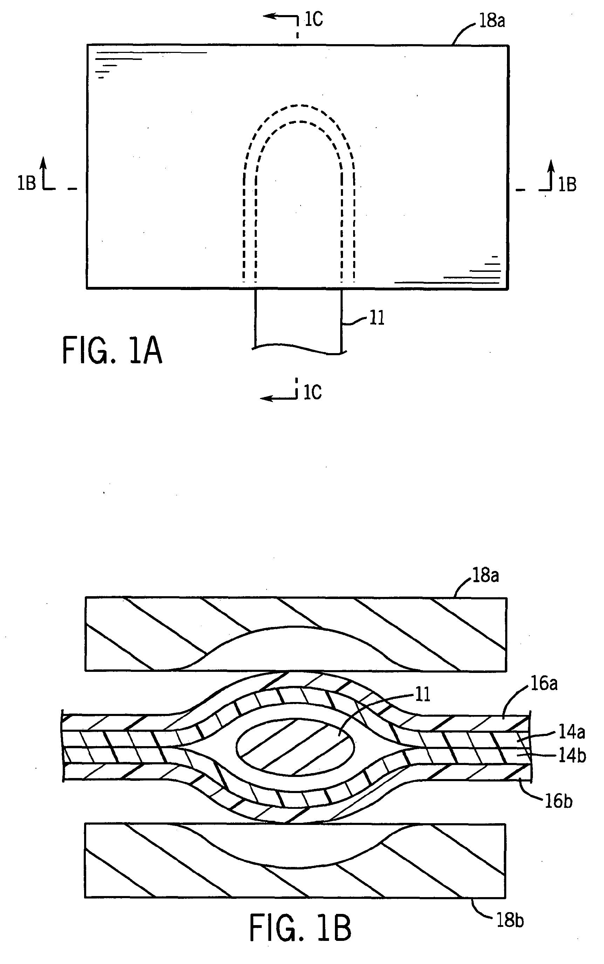

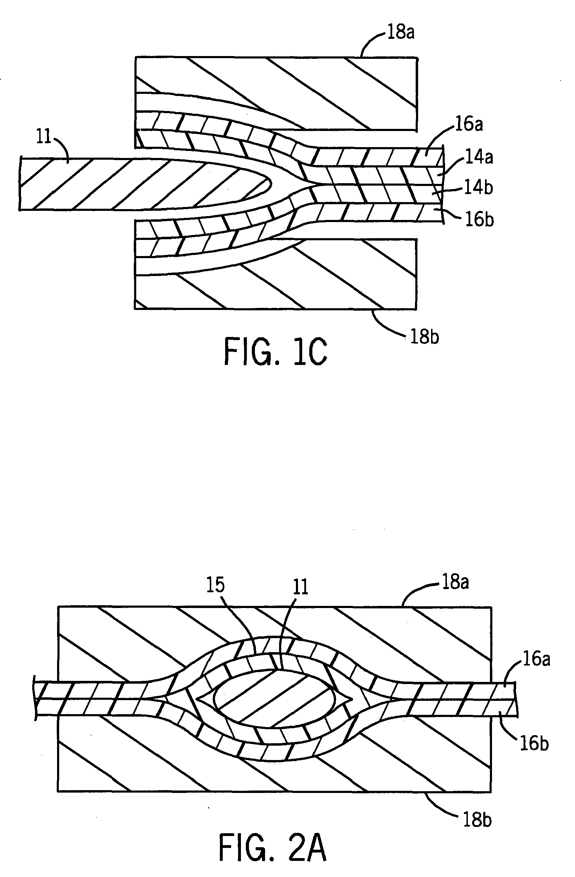

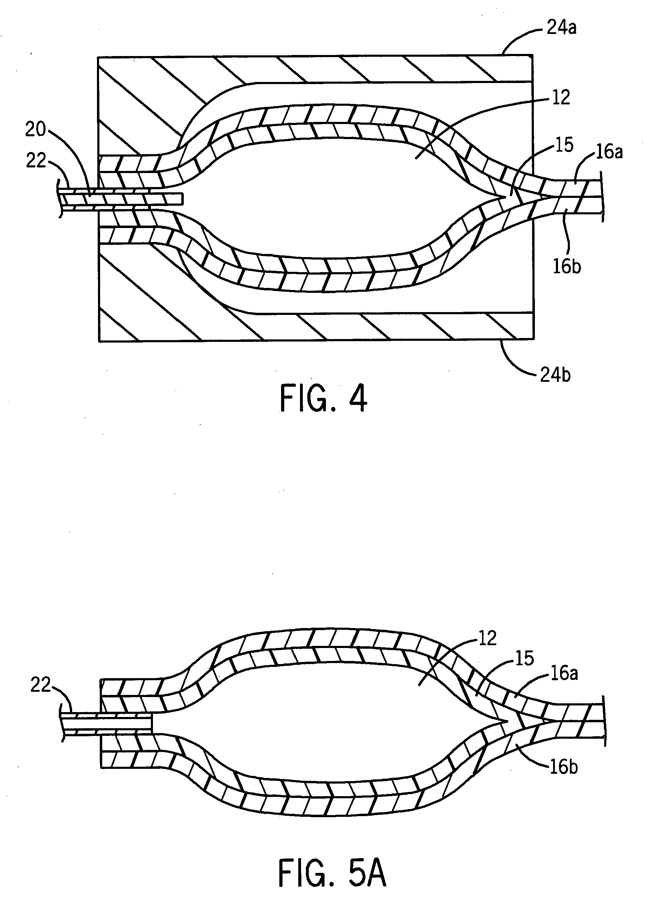

According to embodiments, with reference to Figures 1A, 1B, and 1C, a process for generating microwalled encapsulation balloons may include layering at least two membrane layers 14a and 14b around a paddle insert 11. The paddle insert 11 may define the shape and geometry of an interior portion 12 of the resultant encapsulation balloon 26 (see Figure 4). For example, membrane layers 14a and 14b may be compressed to conform to paddle insert 11. According to embodiments, paddle insert 11 may be of a material that provides selective removal of paddle insert 11 after compression of membrane layers 14a and 14b thereon. Paddle insert 11 may also provide heat and stress resistance. For example, the melting point of the insert may be above that of the porous membrane, such that heat applied at temperatures between the melting point of the porous membrane and the melting point of the insert may cause the porous membrane to partially or completely melt, compress, and conform, without analogous response from the insert. For example, paddle insert 11 may be of a polyimide material, stainless steel, or other rigid or semi-rigid material.

According to embodiments, membrane layers 14a and 14b may be a porous membrane of a biocompatible polymer, such as PTFE, ePTFE, FEP, or another plastic or polymer. According to embodiments, membrane layers 14a and 14b may be porous or otherwise not fully compressed to maximum density. In this low density state, membrane layers 14a and 14b are pliable, compliant, and capable of being compressed to a high density state.

According to embodiments, at least a portion of each of membrane layers 14a and 14b may cover paddle insert 11, such that compression of membrane layers 14a and 14b results in conformity to paddle insert 11, forming an interior portion. According to embodiments, at least a portion of each of membrane layers 14a and 14b may be in contact with each other, such that compression of membrane layers 14a and 14b results in a fusion of membrane layers 14a and 14b around at least a portion of paddle insert 11.

According to embodiments, support layers 16a and 16b may be provided on outer sides of membrane layers 14a and 14b, respectively, as shown in Figures 1B and 1C. Support layers 16a and 16b provide a buffer between corresponding pairs of paddle dies 18a and 18b.

According to embodiments, dies and diecutting procedures used with the processes and devices disclosed herein may include flat bed die cutting or rotary die cutting devices, systems, and procedures. For example, layers may be provided to flat bed die cutting devices, as illustrated in the Figures. By further example, rotary die cutting may be performed (not shown), wherein dies are applied to a rotary press. In a rotary die process, support layers 16a and 16b may be optional.

According to embodiments, support layers 16a and 16b may be a releasing agent of a material that provides selective removal of support layers 16a and 16b from membrane layers 14a and 14b. The releasing agent may also provide resistance to character changes, such as melting, under heat and stress conditions. For example, the melting point of the releasing agent may be above that of the porous membrane, such that heat applied at temperatures between the melting point of the porous membrane and the melting point of the releasing agent may cause the porous membrane to partially or completely melt, compress, and conform, without analogous response from the releasing agent. The releasing agent may also transmit heat and stress applied thereto. For example, the releasing agent may be of a flexible polyimide material.

According to embodiments, paddle dies 18a and 18b may be provided on outer sides of support layers 16a and 16b, respectively, as shown in Figures 1B and 1C. Paddle dies 18a and 18b may be of a solid material and configured to apply pressure to support layers 16a and 16b, membrane layers 14a and 14b, and paddle insert 11. As shown in Figures 1B and 1C, paddle dies 18a and 18b may be shaped and contoured to define customizable shapes of a resultant encapsulation balloon 26.

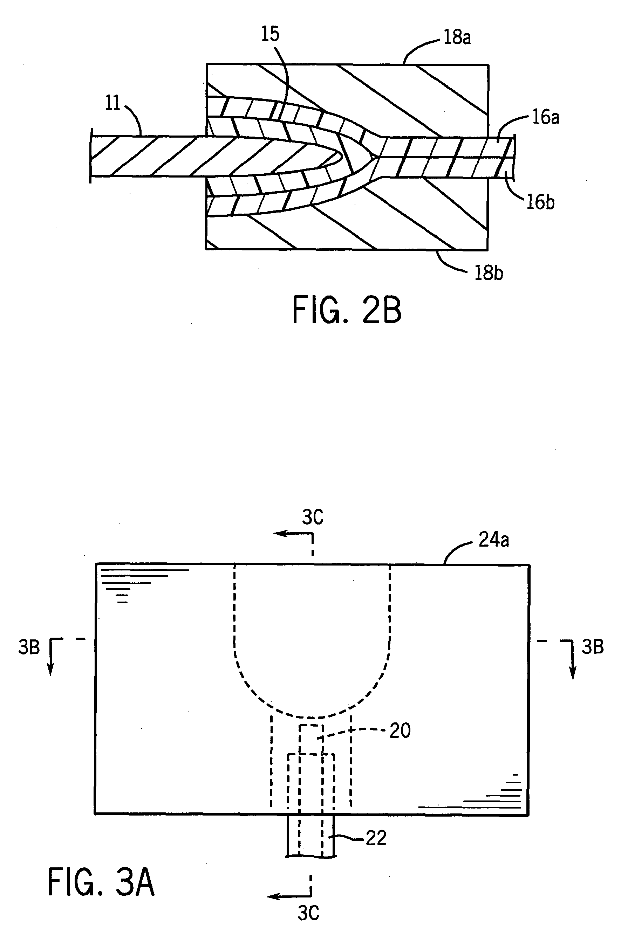

According to embodiments, as shown in Figures 2A and 2B, paddle dies 18a and 18b may be pressed together to define the shape of membrane layers 14a and 14b. For example, pressing paddle dies 18a and 18b together may result in conforming portions of membrane layers 14a and 14b to paddle insert 11 to form an interior portion 12 and opening 13 or the resultant encapsulation balloon 26. According to embodiments, the portions of membrane layers 14a and 14b conformed to paddle insert 11 may compress from its original low density state to a medium or high density state. For example, membrane layers 14a and 14b may be porous in a low density state, as with ePTFE. In a medium density state, membrane layers 14a and 14b may be more compressed than in a high density state, but still retain some porosity. In a medium density state, membrane layers 14a and 14b may further be less compressed than in a high density state. In a high density state, membrane layers 14a and 14b may be at or substantially near the maximum density attainable for the material used.

According to embodiments, the amount of pressure provided while pressing paddle dies 18a and 18b together may be any pressure sufficient to achieve the conformity desired, based on the shape and geometry of paddle dies 18a and 18b together and paddle insert 11.

According to embodiments, the resultant density of the portions of membrane layers 14a and 14b conformed to paddle insert 11 may be controlled and selected based on desired characteristics for subsequent use of the resultant encapsulation balloon. For example, where tissue ingrowth is to be facilitated, the portions of membrane layers 14a and 14b conformed to paddle insert 11 may be uncompressed by paddle dies 18a and 18b and remain in an unchanged low density state, or may be partially compressed by paddle dies 18a and 18b and achieve a medium density state. Alternatively, the portions of membrane layers 14a and 14b conformed to paddle insert 11 may achieve a high density state to provide other characteristics. The density may be controlled by the chosen relative geometries and shapes of paddle dies 18a and 18b and paddle insert 11. Determination of density to be achieved may be governed by consideration of the application of the encapsulation balloon 26, the fill material used, the environment of the emplacement location, among others which are known to those skilled in the art and considered within the scope of the present disclosure.

According to embodiments, pressing paddle dies 18a and 18b together may result in conforming membrane layers 14a and 14b to each other at portions that extend beyond paddle die 11. According to embodiments, paddle dies 18a and 18b may be configured to cause portions of membrane layers 14a and 14b that extend beyond paddle die 11 to achieve a high density state. Such an action may cause membrane layers 14a and 14b become an integrated balloon membrane 15 or otherwise adhere to each other around at least a portion of interior portion 12.

According to embodiments, heat may be applied as paddle dies 18a and 18b are pressed together to facilitate the compression of membrane layers 14a and 14b. For example, membrane layers 14a and 14b may be heated to or near the melting point of the material used. Where PTFE and polyimide is used, membrane layers 14a and 14b may be heated to between about 320°C and about 400°C.

According to embodiments, after paddle dies 18a and 18b and paddle insert 11 are removed, support layers 16a and 16b remain with balloon membrane 15 (resulting from joining of membrane layers 14a and 14b) defining interior portion 12 and opening 13. According to embodiments, pressing paddle dies 18a and 18b together may result in cohesion of balloon membrane 15 to support layers 16a and 16b. Upon completion of the pressing or heating step(s) and removal of paddle dies 18a and 18b, forces may provide continued cohesion between balloon membrane 15 and support layers 16a and 16b. Such forces may include electrostatic, Van der Waals, hydrogen bonding, interlacing, friction, surface tension, capillary forces, among others.

According to embodiments, cohesive forces between balloon membrane 15 and support layers 16a and 16b may allow balloon membrane 15 to retain its form achieved during the pressing or heating step(s). Such support may be utilized to perform additional steps after the application of paddle dies 18a and 18b. For example, support layers 16a and 16b may maintain the shape of interior portion 12 in the absence of paddle insert 11. By further example, support layers 16a and 16b may maintain the shape of opening 13 in the absence of paddle insert 11.

According to embodiments, support layers 16a and 16b may be selectively removable from balloon membrane 15 when the support provided is no longer needed or desired. Removal of support layers 16a and 16b from balloon membrane 15 may be easily achieved when certain materials are used, such as where relatively weak cohesive forces bind support layers 16a and 16b to balloon membrane 15.

According to embodiments, where support layers 16a and 16b are of a polyimide material, support to balloon membrane 15 is enhanced by the tensile strength of the polyimide material as compared to the pliability of membrane materials, such as PTFE and FEP.

According to embodiments, encapsulation balloon 26 may be coupled to a cannula or other surgical or medical device. As used herein, "cannula" refers to any device used to facilitate delivery of encapsulation balloon 26 to a patient. According to embodiments, as shown in Figures 3A to 3C, cannula 22 may be inserted into opening 13 of encapsulation balloon 26, such that a distal end of cannula 22 extends through opening 13 into interior portion 12. Cannula 22 may be of stainless steel or other material conducive to surgical or medical procedures. Cannula 22 may be of a material that is conducive to bonding with a portion of balloon membrane 15. Cannula 22 may be coated with materials to facilitate coupling with or decoupling from balloon membrane 15.

According to embodiments, cannula 22 may have a lumen through which dowel 20 may be advanced. Dowel 20 may extend beyond the distal end of cannula 22 such that it provides support beyond cannula 22 to maintain an opening that extends beyond the portion of balloon membrane 15 that encloses cannula 22. Dowel 20 may be any structure capable of substantially sustaining its form under stress and heat conditions. Dowel 20 may be of a material that is resistant to permanent bonding with balloon membrane 15. For example, dowel 20 may be of a polyimide material.

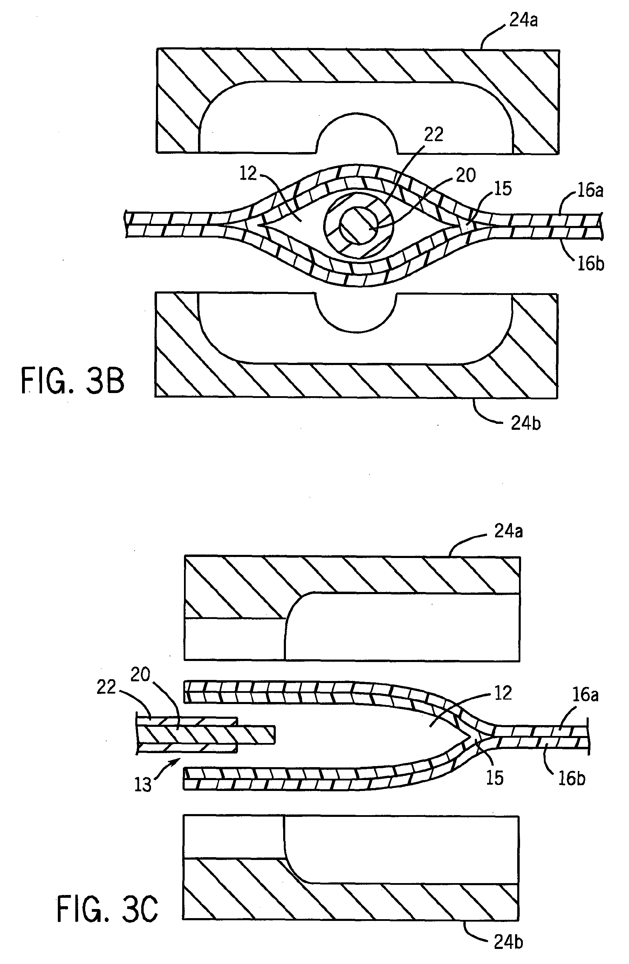

According to embodiments, cylinder dies 24a and 24b may be provided on outer sides of support layers 16a and 16b, respectively, as shown in Figures 3B and 3C. Cylinder dies 24a and 24b may be of a solid material and configured to apply pressure to support layers 16a and 16b, balloon membrane 15, and cannula 22.

According to embodiments, cylinder dies 24a and 24b may be configured to couple balloon membrane 15 to cannula 22. Cylinder dies 24a and 24b may be shaped and contoured to define customizable shapes of a resultant encapsulation balloon 26 and modify opening 13. For example, as shown in Figures 3B and 3C, portions of cylinder dies 24a and 24b may form a section that is more narrow near the portion of balloon membrane 15 that overlaps with cannula 22 or dowel 20. Such a configuration causes balloon membrane 15 to conform to cannula 22 or dowel 20 without collapsing the remainder of interior portion 12 of balloon membrane 15.

According to embodiments, as shown in Figure 4, cylinder dies 24a and 24b may be pressed together to define the shape of a portion of balloon membrane 15. For example, pressing cylinder dies 24a and 24b together may result in conforming portions of balloon membrane 15 to cannula 22 or dowel 20. According to embodiments, characteristics of cannula 22 may facilitate coupling of balloon membrane 15 to cannula 22, as disclosed herein. For example, cannula 22 may be brushed to provide increased surface area for bonding. According to embodiments, characteristics of dowel 20 may prevent permanent bonding with balloon membrane 15, as disclosed herein.

According to embodiments, where more than one material is used to form balloon membrane 15, customized heating may be provided while pressing cylinder dies 24a and 24b together. For example, the portion of balloon membrane 15 enclosing cannula 22 may be of a first material (e.g., FEP), and the remainder of balloon membrane 15 may be of a second material (e.g., PTFE). Where the first and second materials have different melting points, a temperature gradient may be provided across the transition between the first material and the second material through cylinder dies 24a and 24b. According to embodiments, heat may be applied in greater amounts to the material with a higher melting point, and the heat may be allowed to be conducted along cylinder dies 24a and 24b until it reaches the material with a lower melting point, thereby providing a lower temperature. Such temperature gradients may be achieved and controlled by those skilled in the art knowing the characteristics of the materials provided. For example, the temperature may be maintained below about 350°C for PTFE and below about 300°C for FEP using a temperature gradient.

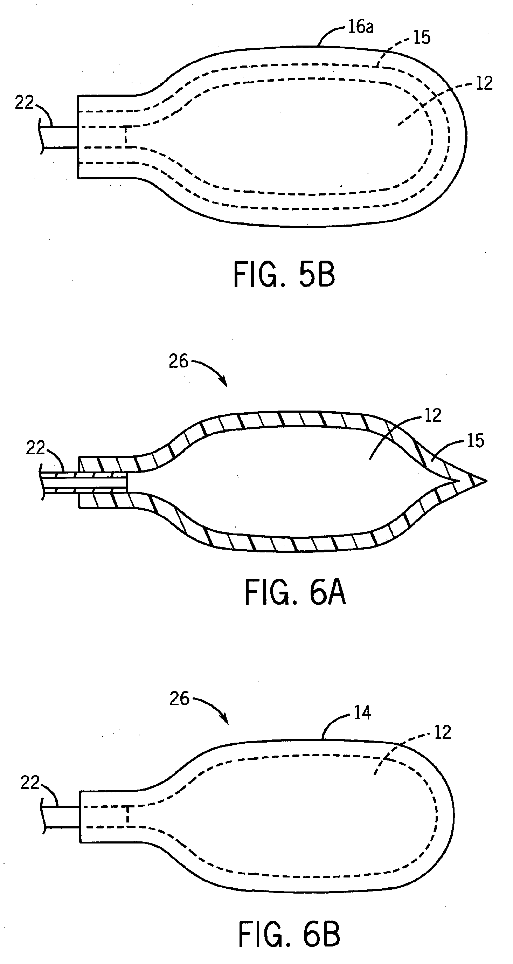

According to embodiments, cylinder dies 24a and 24b may be removed, leaving the resultant balloon membrane 15, cannula 22, and support layers 16a and 16b. As shown in Figure 5A and 5B, portions of balloon membrane 15 and support layers 16a and 16b may be cut out of the total amount of materials used. For example, portions of balloon membrane 15 extending beyond interior portion 12 may be cut out, reducing the amount of excess material in the resultant encapsulation balloon 26. According to embodiments, a variety of methods and devices may be utilized to cut out portions of balloon membrane 15. For example, die cutting, laser cutting, or other programmable, precise, or high-throughput cutting methods may be employed. According to embodiments, support layers 16a and 16b may be removed prior to or remain attached during the cutting process. Where polyimide materials are used for support layers 16a and 16b, support layers 16a and 16b may provide additional support during cutting. Support layers 16a and 16b of crystalline polyimide materials would likewise be compatible with cutting procedures.

According to embodiments, as shown in Figure 6A and 6B, support layers 16a and 16b may be removed, revealing encapsulation balloon 26 coupled to cannula 22. Interior portion 12 of encapsulation balloon 26 may be reduced by deflation through the lumen of cannula 22. Upon delivery to an emplacement site in a patient, encapsulation balloon 26 may be inflated through the lumen of cannula 22 or other devices therein.

According to embodiments, other arrangements for coupling encapsulation balloon 26 to cannula 22 may be achieved. For example, portions of balloon membrane 15 may be disposed within the lumen of cannula 22, with dowel 20 within balloon membrane 15 and pressing balloon membrane 15 against the lumen walls of cannula 22.

Such products produced by the processes disclosed may be configured for a variety of purposes, with components customized the intended purposes. For example, a microwalled encapsulation balloon may be configured to be implanted into tissue of a living organism.

This application cites U.S. Patent No. 6,942,677, issued September 13, 2005; U.S. Patent No. 6,360,749, issued March 26, 2002; U.S. Patent Publication No. 2004/0005295, published January 8, 2004; U.S. Patent Publication No. 2004/0106896, published June 3, 2004; U.S. Patent Publication No. 2004/0180043, published September 16, 2004; U.S. Patent Publication No. 2005/0271631, published December 8, 2005; U.S. Patent Publication No. 2008/0065046, published March 13, 2008; U.S. Patent Publication No. 2008/0065047, published March 13, 2008; U.S. Patent Publication No. 2008/0065048, published March 13, 2008; U.S. Patent Publication No. 2008/0069801, published March 20, 2008; and U.S. Patent Publication No. 2008/0269720, published October 30, 2008.

Despite continuous improvements in prevention, diagnosis, and treatment of cardiovascular diseases, the prevalence of congestive heart failure (CHF) continues to increase worldwide. With an aging and more sedentary population, CHF is one of the most prevalent chronic diseases in industrialized societies and represents the most common reason for hospitalization in patients who are 65 years and older. In the United States alone, about 5 million Americans are currently affected with the number of patients expected to double by the year 2020 - thus becoming the most common form of heart disease.

The economic impact currently surpasses an estimated $29 billion per year and is expected to rise accordingly. Approximately 60 percent of the cost is taken up by hospitalization-related expenses. Since there is no known cure for CHF (except for a heart transplant), patients are managed with a variety of pharmacologic agents and modification of lifestyle. Ultimately, if patients reach the most advanced stage of heart disease, 50 percent of them die within a year.

CHF occurs when the heart's output or pumping capacity is insufficient for the body's needs. The cause for this deficiency may include several underlying diseases such as ischemia, hypertension, obesity and valvular disease. Irrespective of the underlying cause of CHF, in a large percentage of patients, the left ventricle begins to dilate and enlarge, the muscle wall becomes thinner and the chamber looses its proper shape in an effort to compensate pumping capacity. Once it is set in motion, this negative chain of events results in dilated cardiomyopathy (DCM) and cannot be reversed.

Clinical treatment primarily involves the use of various pharmaceuticals aimed at improving symptoms, reducing the workload of the heart and slowing down the progression of the disease. Stem cells are being used experimentally with marginal benefits to regenerate heart muscle. A number of invasive devices and pump assist devices are being developed with mixed success aimed at slowing the disease progression.

Devices made using methods according to embodiments of the present disclosure may prevent or reverse the progression of congestive heart failure (CHF) in patients who have an enlarged left ventricle as a result of mitral valve regurgitation, ischemia, dilated cardiomyopathy and/or other disorders.

Devices made using methods according to embodiments of the present disclosure include a biopolymer that is injected using an applicator, directly into strategic areas of the left ventricle muscle during open heart surgery, catheter-based procedures, or any other interventional procedure. As it is injected, the biopolymer forms bodies that remain in the heart muscle as permanent implants. These implants provide several beneficial functions: (i) they thicken the muscle wall, (ii) reduce chamber size, (iii) decrease local muscle wall stress, and based on the positioning of the implants, (iv) allow for a re-shaping of the dilated ventricle to impart it a healthier form. Based on the results of studies, these functions lead to a global improvement of the heart as measured by ejection fraction, systolic and diastolic pressures and other biomarkers of stress. The positive effect of the biopolymer implants is measurable within hours of the procedure and remains over the long term.

According to , the volume augmentation devices described in the present disclosure may be used for tissue bulking in a variety of circumstances, depending on the need. For example: in gastroenterology, wherein increasing the volume of tissue at the gastro-esophageal junction can be used to treat gastro-esophageal reflux disease, and increasing the thickness of the gastric mucosa to decrease the volume of the stomach to treat morbid obesity; in urology, where placing filler radially around the urethra at the neck of the urinary bladder can ameliorate incontinence; and in cardiology, whereby tissue filler may be placed in the ventricular wall to decrease the volume of the left ventricular chamber to treat heart failure, or in the pericardial space to place pressure on the outside of the heart, also intended to decrease the volume of the heart chambers and thereby treat heart failure; and in other applications well known to those skilled in the art. In any of these clinical applications, the tissue-filling device may be combined with any number of other bioactive substances which may be released from the filler itself over time, or be injected concurrently.

According to , one use of the devices and systems of the present disclosure is in the field of cosmetic plastic surgery wherein the system is used for augmentation in the dermis or subdermis to treat skin contour deficiencies caused by various conditions, including aging, environmental exposure, weight loss, child bearing, surgery, disease such as acne and cancer, or combinations thereof, or for beauty enhancement. The tissue augmentation method of the present disclosure is particularly suitable for treating frown lines, worry lines, wrinkles, crow's feet, facial scars, or marionette lines, or to augment facial features such as the lips, cheeks, chin, nose or under the eyes. Treatment of a patient may consist solely of using a tissue-filing device, or the tissue-filling device may be used as part of additional cosmetic surgery such as a face or brow lift. The characteristic of change from first configuration to second configuration makes the tissue-filling device desirable for use in endoscopic surgery. The tissue augmentation device may also be used for breast augmentation, and regions of the body that need volume enlargement during reconstructive plastic surgery, such as after trauma or tumor resection.

The present disclosure addresses those aspects of designing an optimized composition for tissues that need to be repaired. The injectable composition of this disclosure is also suitable for the treatment of many tissue conditions such as augmentation and strengthening of tissue in patients. Other than the plastic surgery or reconstructive surgery, tissue fillers can be used to correct aphonia or dysphonia caused by paralysis of the vocal cords, to correct defect or injury, to the augmentation of hypoplastic breast, to the augmentation of scar tissue, to the treatment of urological disorders (e.g. urinary incontinence), to the treatment of incompetent anal sphincters, to the treatment of vesicoureteral reflux, and to the treatment of gastric fluid reflux by endoscopic or subcutaneous injection of biocompatible hollow particular implants into the submucosal or dermal tissue. Since the disclosure is closely related to the treatment of soft tissue augmentation, it will be described in details by reference hereto.

The fill used in conjunction with an encapsulation balloon may be of a biologically compatible natural or synthetic material ("biomaterial") that is flowable under pressure through a lumen and therefore injectable into the body. Once within the encapsulation balloon *in situ*, the biomaterial may remain in the same flowable state, or may transition from the flowable state to a non-flowable state such as a solid or a semi-solid gel material. The biomaterial may also swell during and/or after the transition. Suitable biomaterial includes a wide variety of substances typically used in medicine and commonly classified as synthetic or natural polymers, copolymers, biopolymers, silicone biomaterials, hydrogels, and composites. Examples of polymers include (a) human or animal-derived such as fibrin glue, collagen, hyaluronic acid, and chitosans; (b) plant-derived polymers such as alginates or biocompatible liquids and gels derived from starch, sugar, and cellulose; (c) lactic acid - based gels and liquids; and (d) synthetically derived materials such as Polyethylene Glycol ("PEG"). The term "biomaterial" may refer to only one biomaterial or a combination of biomaterials, and does not require or preclude the presence of one or more bioreactive components such as (a) cells (stem cells, fibroblasts, skeletal cells, and others); (b) proteins and peptides from the families of growth factors, cytokines, and chemokines; (c) plasmids or genes; (d) natural or engineered binding sites such as RDG binding sites and antibodies and antibody constructs; (e) various pharmaceutical compositions; (f) any other therapeutically beneficial materials; or (g) any combination of the foregoing. Suitable biomaterial also includes silicone and saline.

The fill material and/or the encapsulation balloon may be radiopaque or may contain radiopaque markers to facilitate visualization within the tissue.

According to , the encapsulation balloon may be sealable at an end upon delivery into a patient. Many different techniques are suitable for sealing the encapsulation balloon, including welding by ultrasound, radio frequency, or thermal energy, bonding by the use of adhesive, and mechanical closure by twisting or clamping. An example of a ultrasound catheter apparatus that may be adapted for the purposes described herein is disclosed in US Patent No. 6,942,677 issued September 13, 2005 to Henry Nita et al., Where adhesive bonding is desired, a suitable adhesive is a biologically compatible material that flows through one or more lumen for deposition into the neck of the capsule. The adhesive adheres to the encapsulation balloon and forms a seal that seals the neck. In some implementations, the adhesive may be highly viscous and may generally remain highly viscous, while in other exemplary implementations, the adhesive may transition from a flowable to a non-flowable state. The adhesive may be deposited over the capsule neck in the flowable state, and then transition into the non-flowable state to form the seal. Examples of suitable adhesive include medical glues such as the cyanoacrylates and sulfacrylates used in surgery.

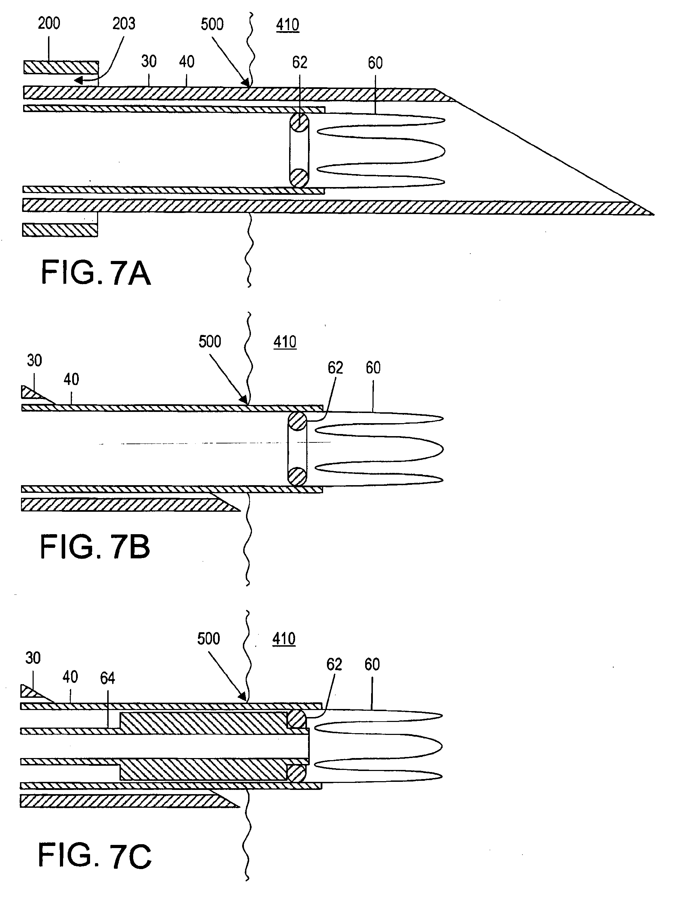

Figures 7A to 7E, 8A to 8D and 9A to 9D show portions of an illustrative emplacement apparatus 10 for emplacing a capsule 60, such as one produced by the process disclosed herein according to embodiments of the present disclosure, generally proximate the distal end thereof in various operational conditions.

As illustrated in Figure 7A, the sheath 200 is tubular and defines a sheath lumen 203. The needle 30 and the membrane introducer tube 40 are illustrated as received in part within the sheath lumen 203 and deployed distally so that portions of the needle 30 and the membrane introducer tube 40 extend distal of the distal end of the sheath 200 in this operational condition. In other operational conditions (not shown) the needle 30 and the membrane introducer tube 40 may be positioned entirely within the sheath lumen 203 such that the tip of the needle 30 and the distal end of the membrane introducer tube 40 are contained.

As further illustrated in Figure 7A, the needle 30 defines a needle lumen. Illustratively, the tip of the needle 30 is beveled to form a cutting edge for ease of penetration into tissue 410. The needle 30 is inserted into the tissue 410 at the injection site 500, and the tissue 410 is generally biased about the outer surface of the needle 30.

The membrane introducer tube 40 defines a lumen, and includes at its distal end an encapsulation membrane 60 in a collapsed position. The encapsulation membrane 60 is collapsed into a number of folds and is secured to the inner surface of the membrane introducer tube 40 generally in the area of seal 62. The membrane introducer tube 40 is moveably received within the lumen of the needle 30 such that the distal end of the membrane introducer tube 40 and the tip of the needle 30 are positionable with respect to one another. In the operational condition illustrated in Figure 7A, the distal end of the membrane introducer tube 40 is positioned within the lumen of the needle 30 and near the tip thereof. When the needle 30 penetrates the injection site 500 and is advanced into the tissue 410, the encapsulation membrane 60 is brought into position within the tissue 410.

In the operational condition of the emplacement apparatus 10 shown in Figure 7B, the needle 30 is retracted proximally with respect to the distal end of the membrane introducer tube 40 to withdraw it from the tissue 410. The position of the distal end of the membrane introducer tube 40 is generally maintained while the needle 30 is being retracted from the tissue 410, so that the encapsulation membrane 60 remains in the tissue 410 in a collapsed condition.

In the operational condition of the emplacement apparatus 10 shown in Figure 7C, a fill tube 64 is advanced through the lumen of the membrane introducer tube 40 until it presses up against and engages the seal 62.

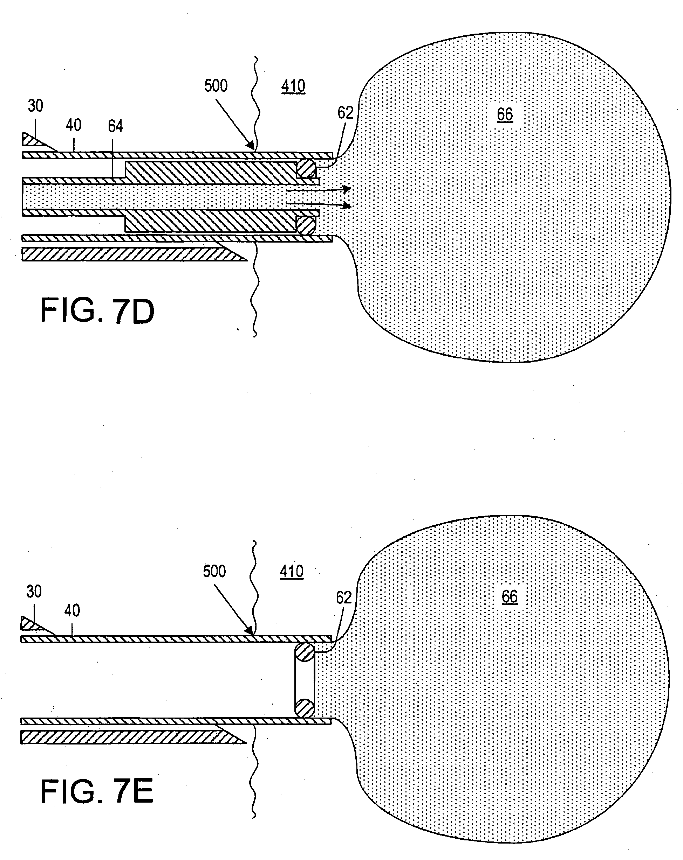

In the operational condition of the emplacement apparatus 10 shown in Figure 7D, biomaterial is injected through the fill tube 64 to expand the encapsulation membrane 60 into capsule 66.

In the operational condition of the emplacement apparatus 10 shown in Figure 7E, the fill tube 64 is withdrawn from the membrane introducer tube 40.

While sealing of the capsule 66 is generally advantageous, it may not be necessary when the biomaterial contained in the capsule 66 becomes sufficient rigid or semi-rigid and other undesirable effects of exposing the biomaterial to body fluids are not troublesome. If the capsule 66 is not to be sealed, the capsule 66 is detached from the membrane introducer tube 40 using any suitable technique, and the membrane introducer tube 40 is withdrawn from the tissue 410 to leave the capsule 66 within the tissue 410. Suitable techniques for detaching the capsule 66 from the membrane introducer tube 40 include twisting the membrane introducer tube 40 to rupture the neck of the capsule 66 or dislodge the seal 62 from the lumen of the membrane introducer tube 40, advancing a plunger through the lumen of the membrane introducer tube 40 and against the seal 62 to push the seal 62 and the neck of the capsule 66 out of the distal end of the membrane introducer tube 40, and forcibly withdrawing the membrane introducer tube 40 from the tissue 410 so that the seal 62 and the neck of the capsule 66 are pulled out of the membrane introducer tube 40 as the capsule 66 is held in place within the tissue 410, which is biased against it. Since various techniques may be used to secure the encapsulation membrane 60 and the seal 62 to the lumen of the membrane introducer tube 40, including press-fit, adhesive, and welding, the choice of securing technique and detachment technique are preferably coordinated.

If the capsule 66 need not be sealed, an alternative technique may be practiced in which the encapsulation membrane 60 is filled directly through the lumen of the membrane introducer tube 40. In this alternative technique, the seal 62 and the fill tube 64 need not be used.

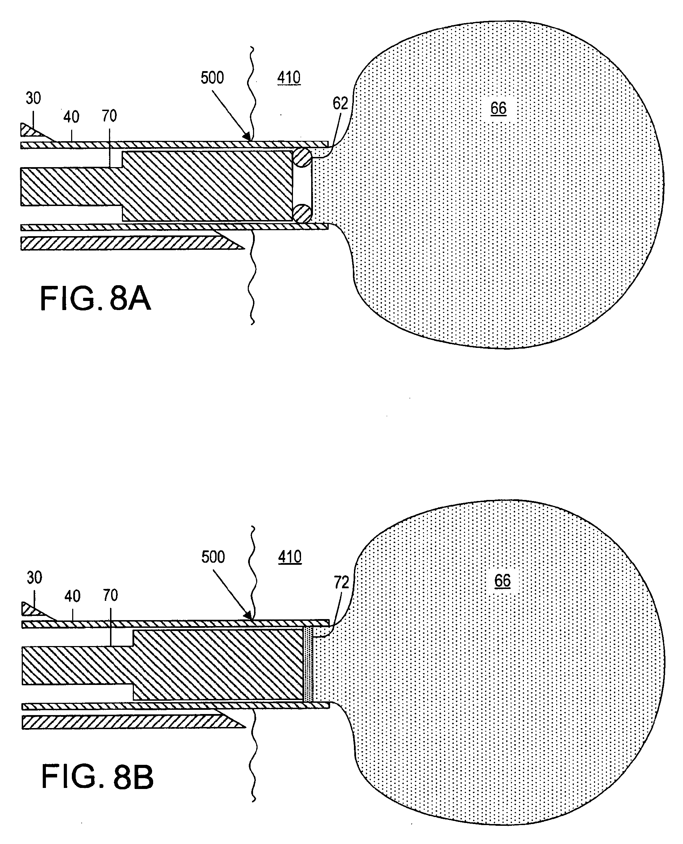

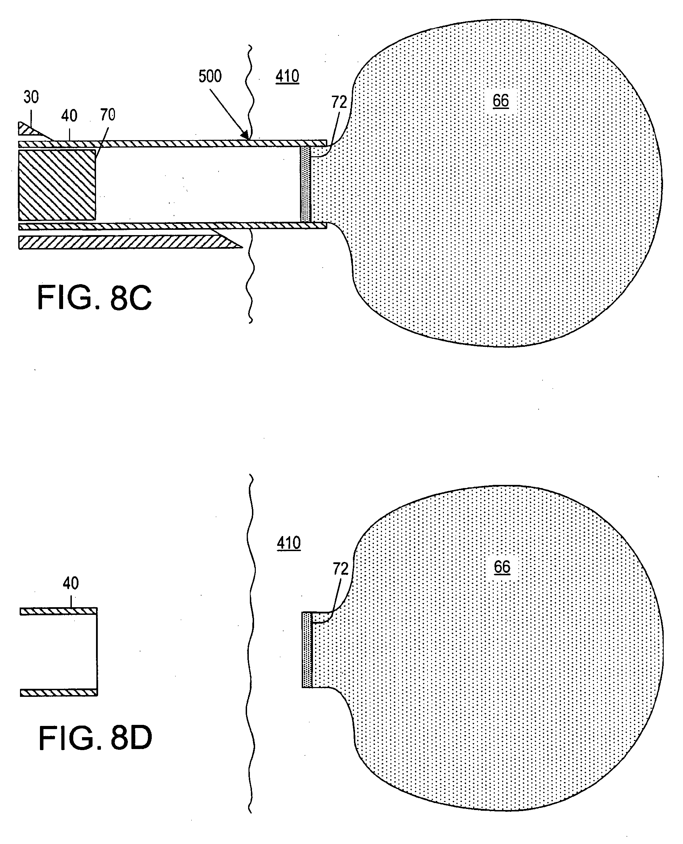

If one desires to seal the capsule 66, an ultrasonic welding technique as operationally shown in Figures 8A to 8D may be used. As shown in Figure 8A, a suitable elongated ultrasonic welding device is advanced through the lumen of the membrane introducer tube 40 until the distal welding tip 70 of the device engages the seal 62 and is slightly biased against the seal 62. The seal 62 is made of a suitable material such as a pliable biologically compatible plastic that softens and flows when subjected to ultrasonic energy, so that upon activation of the ultrasonic welding device as shown in Figure 8B, the seal 62 flows to form plug 72. The welding tip 70 is withdrawn from the plug 72 as shown in Figure 8C, leaving the plug 72 in place in the neck of the capsule 66. When the capsule 66 as sealed by the plug 72 is detached from the membrane introducer tube 40 as described previously, and the membrane introducer tube 40 is withdrawn from the tissue 410 as shown in Figure 8D, the sealed capsule 66 remains in place within the tissue 410.

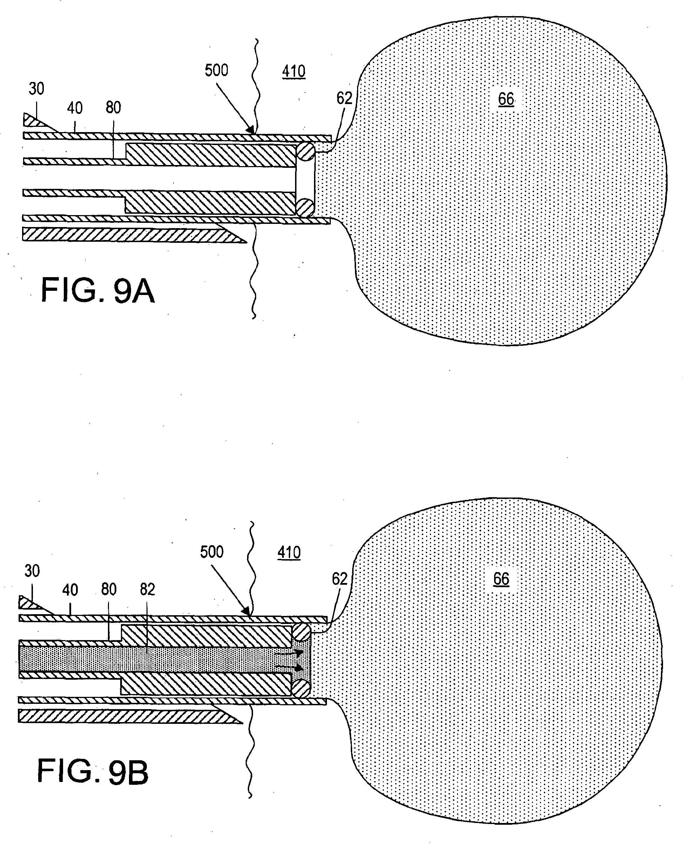

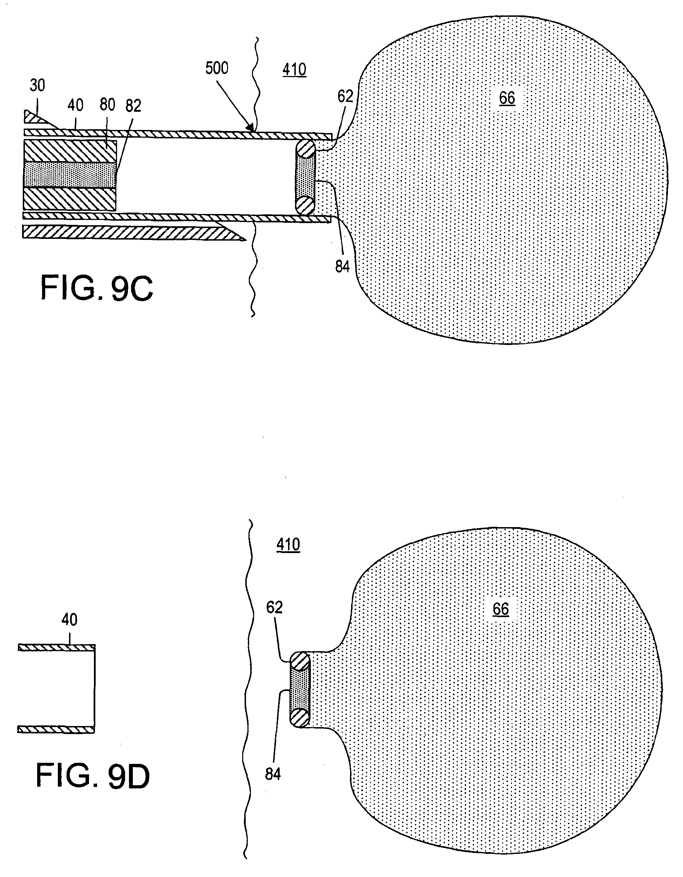

If one desires to seal the capsule 66, an adhesive sealing technique as operationally shown in Figures 9A to 9D may be used. As shown in Figure 9A, an adhesive applicator tube 80 is advanced through the lumen of the membrane introducer tube 40 until the distal tip of the applicator tube 80 engages the seal 62. As shown in Figure 9B, a flowable adhesive 82 is injected into the neck of the capsule 66 so as to fill the neck of the capsule 66. The adhesive 82 is selected to have good adhesion with the seal 62, and may also have good adhesion to the surface of the biomaterial exposed within the neck of the capsule 66. When the applicator tube 80 is withdrawn as shown in Figure 9C, a plug 84 of the adhesive 82 remains in the neck of the capsule 66 proximate the seal 62. The plug 84 completes the seal of the neck of the capsule 66 to prevent contact between the tissue 410 and the biomaterial within the capsule 66. When the capsule 66 as sealed by the seal 62 and the plug 84 is detached from the membrane introducer tube 40 as described previously, and the membrane introducer tube 40 is withdrawn from the tissue 410 as shown in Figure 8D, the sealed capsule 66 remains in place within the tissue 410.

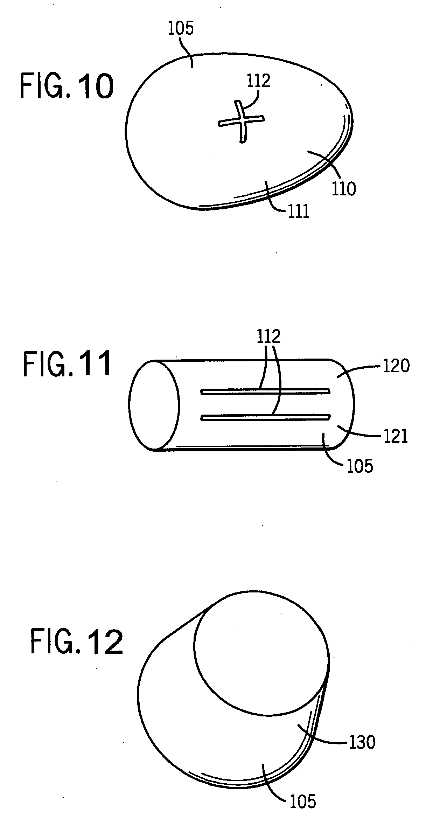

The capsule 66 as illustrated in Figures 8A to 8D and 9A to 9D, is generally spherical in shape. However, other shapes may be used if desired. Figure 10 shows and example of a capsule 110 that has an ovoid shape. Radiopaque markings 112 may be included on the outer surface of the capsule 110 so that the position of the capsule 110 may be located in vivo. Figure 11 shows an example of a capsule 120 that has a cylindrical shape. Radiopaque markings may be included on the outer surface of the capsule 120 to facilitate locating the capsule 120. Figure 12 shows an example of a capsule 130 that has a frustoconical shape.

According to , an illustrative method of operation of the emplacement apparatus 10 is as follows. The distal end of the sheath 200 is inserted into the patient. The needle 30 and the membrane introducer tube 40 are contained within the lumen of the sheath 200 such that the distal end of the emplacement apparatus 10 is generally atraumatic. The sheath distal end 204 is navigated through various bodily passages until generally proximate the injection site 500.

According to , the needle 30 is then deployed with the tip of the needle 30 being inserted into the tissue 410 to a depth that allows for the positioning of the membrane introducer tube 40 within the tissue 410. If not previously in position, the membrane introducer tube 40 with encapsulation membrane 60 in the collapsed position is advanced through the lumen of the needle 30 until positioned at the distal end of the needle 30, at a suitable depth within the tissue 410. Then, the needle 30 is withdrawn from the tissue 410, leaving the encapsulation membrane 60 at the distal end of the membrane introducer tube 40 properly positioned at the desired emplacement site within the tissue 410.

According to , biomaterial is flowed into the encapsulation membrane 60 to inflate the encapsulation membrane 60 from the collapsed condition into an inflated condition at the emplacement site. Optionally, the neck of the encapsulation membrane 60 is sealed to form the capsule 66. The membrane introducer tube 40 is disengaged from the encapsulation membrane 60 and withdrawn from the tissue 410, leaving the capsule 66 in position at the emplacement site.

According to , a kit of parts is disclosed. One or more kits of parts can be envisioned by the person skilled in the art, the kits of parts to perform at least one of the methods herein disclosed. Likewise, directions for use ("DFU") are included and the device may be part of a surgical tray or other packaged accessory set for surgeries. The kit may be a sub-component of a surgical tray.

## Claims

1. A process for generating a microwalled encapsulation balloon, comprising, in combination:
providing a paddle insert having a top side and a bottom side;
placing a balloon membrane further comprising a first membrane layer on at least a portion of the top side of the paddle insert and a second membrane layer on at least a portion of the bottom side of a paddle insert, such that portions of the first membrane layer and the second membrane layer extending beyond the paddle insert are in contact with each other;
providing a first support layer on a top side of the first membrane layer and a second support layer on a bottom side of the second membrane layer;
providing a first paddle die on a top side of the first support layer and a second paddle die on a bottom side of the second support layer; and pressing the first and second paddle dies together to conform the balloon membrane to the paddle insert while applying heat to or near the melting point to the balloon membrane to join membrane layers

2. The process of claim 1, wherein pressing the first and second paddle dies together compresses the portions of the first and second membrane layers beyond the paddle insert to a high density state, whereby first and second membrane layers become an integrated balloon membrane.

3. The process of claim 1, wherein pressing the first and second paddle dies together establishes cohesive force between the first membrane layer to the first support layer and the second membrane layer to the second support layer.

4. The process of claim 1, wherein the heat applied is between a melting point of the balloon membrane and a melting point of the support layers.

5. The process of claim 1, wherein the balloon membrane comprises at least one of PTFE, ePTFE, and FEP.

6. The process of claim 1, wherein the support layer comprises a polyimide and the paddle insert comprises a polyimide.

7. The process of claim 1, further comprising removing the paddle insert, whereby an interior portion of the balloon membrane is provided and accessible through an opening and whereby the first and second support layers maintain the shape of the balloon membrane achieved by pressing the first and second paddle dies together.

8. The process of claim 1, further comprising:
removing the first and second paddle dies; and
coupling the balloon membrane to a cannula by providing a cannula through the opening into the interior portion of the balloon membrane and applying a cylinder die to a portion of the balloon membrane that encloses the cannula.

9. The process of claim 8, further comprising providing a dowel within a lumen of the cannula to the interior portion of the balloon membrane, such that the dowel extends beyond the distal end of the cannula, whereby the dowel maintains the opening of the interior portion of the balloon membrane as the cylinder die is applied.

10. The process of claim 1, further comprising removing the first and second support layers from the balloon membrane.

## Patentansprüche

1. Verfahren zum Erzeugen eines mikrowandigen Einkapselungsballons, das, in Kombination, Folgendes umfasst:
das Bereitstellen eines Schaufeleinsatzes, der eine obere Seite und eine untere Seite hat,
das Anordnen einer Ballonmembran, die ferner eine erste Membranschicht auf wenigstens einem Abschnitt der oberen Seite des Schaufeleinsatzes und eine zweite Membranschicht auf wenigstens einem Abschnitt der unteren Seite eines Schaufeleinsatzes umfasst, derart, dass sich Abschnitte der ersten Membranschicht und der zweiten Membranschicht, die sich über den Schaufeleinsatz hinaus erstrecken, in Berührung miteinander befinden,
das Bereitstellen einer ersten Trägerschicht auf einer oberen Seite der ersten Membranschicht und einer zweiten Trägerschicht auf einer unteren Seite der zweiten Membranschicht,
das Bereitstellen eines ersten Schaufelwerkzeugs auf einer oberen Seite der ersten Trägerschicht und eines zweiten Schaufelwerkzeugs auf einer unteren Seite der zweiten Trägerschicht und das Zusammenpressen des ersten und des zweiten Schaufelwerkzeugs, um die Ballonmembran an den Schaufeleinsatz anzupassen, während Wärme an oder nahe dem Schmelzpunkt auf die Ballonmembran angewendet wird, um die Membranschichten zu verbinden.

2. Verfahren nach Anspruch 1, wobei das Zusammenpressen des ersten und des zweiten Schaufelwerkzeugs die Abschnitte der ersten und der zweiten Membranschicht jenseits des Schaufeleinsatzes bis zu einem Zustand hoher Dichte zusammendrückt, wodurch die erste und die zweite Membranschicht eine integrierte Ballonmembran werden.

3. Verfahren nach Anspruch 1, wobei das Zusammenpressen des ersten und des zweiten Schaufelwerkzeugs eine Kohäsionskraft zwischen der ersten Membranschicht und der ersten Trägerschicht und der zweiten Membranschicht und der zweiten Trägerschicht herstellt.

4. Verfahren nach Anspruch 1, wobei die angewendete Wärme zwischen einem Schmelzpunkt der Ballonmembran und einem Schmelzpunkt der Trägerschichten liegt.

5. Verfahren nach Anspruch 1, wobei die Ballonmembran wenigstens eines von PTFE, ePTFE und FEP umfasst.

6. Verfahren nach Anspruch 1, wobei die Trägerschicht ein Polyimid umfasst und der Schaufeleinsatz ein Polyimid umfasst.

7. Verfahren nach Anspruch 1, das ferner das Entfernen des Schaufeleinsatzes umfasst, wodurch ein innerer Abschnitt der Ballonmembran bereitgestellt wird und durch eine Öffnung zugänglich ist und wodurch die erste und die zweite Trägerschicht die durch das Zusammenpressen des ersten und des zweiten Schaufelwerkzeugs erreichte Form der Ballonmembran aufrechterhalten.

8. Verfahren nach Anspruch 1, das ferner Folgendes umfasst:
das Entfernen des ersten und des zweiten Schaufelwerkzeugs und
das Koppeln der Ballonmembran an eine Kanüle durch das Bereitstellen einer Kanüle durch die Öffnung in den inneren Abschnitt der Ballonmembran und das Anwenden eines Zylinderwerkzeugs auf einen Abschnitt der Ballonmembran, der die Kanüle umschließt.

9. Verfahren nach Anspruch 8, das ferner das Bereitstellen eines Zylinderstifts innerhalb eines Lumens der Kanüle zu dem inneren Abschnitt der Ballonmembran umfasst, derart, dass sich der Zylinderstift über das distale Ende der Kanüle hinaus erstreckt, wodurch der Zylinderstift die Öffnung des inneren Abschnitts der Ballonmembran aufrechterhält, wenn das Zylinderwerkzeug angewendet wird.

10. Verfahren nach Anspruch 1, das ferner das Entfernen der ersten und der zweiten Trägerschicht von der Ballonmembran umfasst.

## Revendications

1. Procédé permettant de créer un ballon d'encapsulation à micro-parois, comprenant, en combinaison, les étapes consistant à :
fournir un insert formant palette présentant un côté supérieur et un côté inférieur ;
placer une membrane de ballon comprenant en outre une première couche de membrane sur au moins une partie du côté supérieur de l'insert formant palette et une deuxième couche de membrane sur au moins une partie du côté inférieur de l'insert formant palette, de telle manière que des parties de la première couche de membrane et de la deuxième couche de membrane s'étendant au-delà de l'insert formant palette sont en contact les unes avec les autres ;
fournir une première couche de support sur un côté supérieur de la première couche de membrane et une deuxième couche de support sur un côté inférieur de la deuxième couche de membrane ;
fournir une première matrice de palette sur un côté supérieur de la première couche de support et une deuxième matrice de palette sur un côté inférieur de la deuxième couche de support ; et presser les première et deuxième matrices de palette ensemble pour que la membrane de ballon épouse l'insert formant palette tout en appliquant à la membrane de ballon de la chaleur au, ou à proximité du, point de fusion pour réunir les couches de membrane.

2. Procédé selon la revendication 1, dans lequel l'étape de pression des première et deuxième matrices de palette ensemble comprime les parties des première et deuxième couches de membrane qui se situent au-delà de l'insert formant palette jusqu'à un état de densité élevée, grâce à quoi les première et deuxième couches de membrane deviennent une membrane de ballon intégrée.

3. Procédé selon la revendication 1, dans lequel l'étape de pression de première et deuxième matrices de palette ensemble crée une force de cohésion entre la première couche de membrane et la première couche de support et entre la deuxième couche de membrane et la deuxième couche de support.

4. Procédé selon la revendication 1, dans lequel la chaleur appliquée est comprise entre un point de fusion de la membrane de ballon et un point de fusion des couches de support.

5. Procédé selon la revendication 1, dans lequel la membrane de ballon comprend au moins un parmi PTFE, ePTFE, et FEP.

6. Procédé selon la revendication 1, dans lequel la couche de support comprend un polyimide et l'insert formant palette comprend un polyimide.

7. Procédé selon la revendication 1, comprenant en outre une étape consistant à retirer l'insert formant palette, grâce à quoi une partie intérieure de la membrane de ballon est fournie et est accessible à travers une ouverture et grâce à quoi les première et deuxième couches de support maintiennent la forme de la membrane de ballon obtenue par pression des première et deuxième matrices de palette ensemble.

8. Procédé selon la revendication 1, comprenant en outre les étapes consistant à :
retirer les première et deuxième matrices de palette ; et
coupler la membrane de ballon à une canule en fournissant une canule à travers l'ouverture jusque dans la partie intérieure de la membrane de ballon et en appliquant une matrice cylindrique sur une partie de la membrane de ballon qui renferme la canule.

9. Procédé selon la revendication 8, comprenant en outre une étape consistant à fournir un goujon au sein d'une lumière de la canule jusqu'à la partie intérieure de la membrane de ballon, de telle manière que le goujon s'étend au-delà de l'extrémité distale de la canule, grâce à quoi le goujon maintient l'ouverture de la partie intérieure de la membrane de ballon lorsque la matrice cylindrique est appliquée.

10. Procédé selon la revendication 1, comprenant en outre une étape consistant à retirer les première et deuxième couches de support de la membrane de ballon.
